# EUROPEAN PATENT SPECIFICATION

(11) **EP 1 556 062 B1**
(45) Date of publication and mention of the grant of the patent: **23.12.2009**
(21) Application number: 03791169.0
(22) Date of filing: 27.08.2003
(51) Int. Cl.: A61K 35/00

(54) **HERBAL EXTRACT COMPRISING A MIXTURE OF SAPONINS OBTAINED FROM SAPINDUS TRIFOLIATUS FOR ANTICONVULSANT ACTIVITY**
KRÄUTEREXTRAKT AUS SAPINDUS TRIFOLIATUS, ENTHALTEND EINE MISCHUNG AUS SAPONINEN MIT KRAMPFLÖSENDER WIRKUNG
EXTRAIT DE PLANTES CONTENANT UN MELANGE DE SAPONINES OBTENUES A PARTIR DU SAPINDUS TRIFOLIATUS A ACTIVITE ANTI-CONVULSIVANTE

(30) Priority: 28.08.2002 IN MU07792002
(43) Date of publication of application: 27.07.2005
(73) Proprietor: Lupin Limited, Mumbai 400 098 Maharashtra (IN)
(72) Inventor: ARORA, Sudershan Kumar, Lupin Ltd. (Res. Park), Taluka Mulshi, 411 042 Pune (IN); SRIVASTAVA, Vandita, Lupin Ltd. (Res. Park), 411 042 Pune (IN); ADDEPALLI, Veeranjaneyulu, Lupin Ltd.(Res. Park), 411 042 Pune (IN); NATESAN, Sridhar, Lupin Ltd. (Res. Park), 411 042 Pune (IN); GOEL, Rajan, Lupin Ltd. (Res. Park), 411 042 Pune (IN)
(74) Representative: Falk, Urs
(86) International application number: PCT/IN2003/000289
(87) International publication number: WO 2004/019960

(56) References cited:
- EP-A- 0 767 177
- CHATURVEDI A.K., ET AL.: "Anti-inflammatory and anticonvulsant properties of some natura plant triterpenoids" PHARMACOLOGICAL RESEARCH COMMUNICATIONS, vol. 8, no. 2, - 1976 pages 199-210, XP009027233
- PATENT ABSTRACTS OF JAPAN vol. 015, no. 012 (C-0795), 10 January 1991 (1991-01-10) & JP 02 262510 A (INABATA KORYO KK), 25 October 1990 (1990-10-25)
- HIGUCHI R ET AL: "SEED SAPONINS OF AKEBIA QUINATA DECNE I. HEDERAGENIN 3-O-GLYCOSIDES" CHEMICAL AND PHARMACEUTICAL BULLETIN, TOKYO, JP, vol. 20, no. 9, 1 July 1972 (1972-07-01), pages 1935-1939, XP002068129 ISSN: 0009-2363
- KANCHANAPOOM T. ET AL.: "Acetylated triterpene saponins from the thai medicinal plant, Sapindus emarginatus" CHEMICAL & PHARMACEUTICAL BULLETIN, vol. 49, no. 9, September 2001 (2001-09), pages 1195-1197, XP001179756 japan

## Description

### FIELD OF THE INVENTION

The present invention relates to a pharmaceutical composition comprising a herbal extract, comprising a mixture of saponins prepared from the pericarp of *Sapindus trifoliatus,* exhibiting useful pharmacological activities, +with binding affinities for the receptor sites viz. GABA-A agonist site, Glutamate-AMPA site, Glutamate-Kainate site, Glutamate-NMDA agonistic site, Glutamate-NMDA glycine (strychnine insensitive) site and Sodium channel (site 2). These receptor sites are known to have major mediatory role in anticonvulsant activity.

The composition with the herbal extract exhibits anticonvulasant activity in Maximal Electroshock Seizure (MES) model. Since anticonvulsants are of particular use in prophylactic treatment of migraine, the present investigation is targeted for the prophylactic treatment of migraine.

The invention further relates to a process for preparation of the herbal extract; isolation of six pure compounds from the mixture of saponins in the aqueous extract; and a pharmaceutical composition comprising the said extract in combination with pharmaceutically acceptable additives.

The invention also relates to the use of the pharmaceutical composition for the manufacture of a pharmaceutical composition for the prophylactic treatment of migraine through anticonvulsant activity by administration of the pharmaceutical composition through the intranasal route.

### BACKGROUND OF THE INVENTION

Convulsion is a type of chronic disorder, which arises due to abnormal neuronal discharges in the Central Nervous System, thus exhibiting seizure activity. Hemicrania, more popularly known as migraine nowadays, is one such chronic episodic disorder characterized by attack of intense pulsatile and throbbing headache, typically unilateral in nature with or without aura. The symptoms associated with the attack are anorexia, nausea, and vomiting and photo-and/or phonophobia. The pathophysiology of migraine is multifactorial and complex in nature.

Several theories/hypotheses have been proposed for explaining the clinical features of migraine. To name a few, these include :
i) The Vasodilation theory, which centers on the involvement of cerebral vasodilatation in development of migraine pain [Wolf, H.G., and Tunis, M.M., Analysis of Cranial Artery Pressure Pulse Waves in Patients with Vascular Headache of the Migraine Type, Trans. Assoc. Am. Physicians, 1952, 65, 240-244; Kimball, R.W. et. al., Effect of Serotonin in Migraine, Neurology, 1960, 10, 135-139].
   Over the years, variations on the vasodilation theory of migraine have been proposed that differ on which cerebral arteries were thought to be involved. More recently, challenges to the this theory have surfaced [Ferrari, M.D. et. al., Arch. Neurol, 1995, 52, 135-139; Goadsby, P. J. and Gundlach, A. L., Localization of 3H-dihydroergotamine Binding Sites in the Cat Central Nervous Systems: relevance to Migraine, Ann. Neurol., 1991, 29(1), 91-9+94].
ii) The Neurological theory, which suggests that migraine arises as a result of abnormal neuronal firing and neurotransmitter release in brain neurons [Pearce, J. M., Migraine: A Cerebral Disorder, Lancet, 1984, 2(8394), 86-89; Welch, K et. al., Central Neurogenic Mechanisms of Migraine, Neurology, 1993, 43 (suppl), S21-25].
iii) The Neurogenic Dural Inflammation theory, which proposes that migraine pain is associated with inflammation and dilation of the meninges particularly the dura, a membrane surrounding the brain [Moskowitz, M.A., Neurogenic Inflammation in the Pathophysiology and Treatment of Migraine, Neurology, 1993, 43(6 suppl 3), S 16-20; Goadsby, P.J. et.al., Release of Vasoactive Peptides in the Extracerebral Circulation of Humans and the Cat during Activation of the Trigeminovascular System, Ann. Neurol., 1988, 23, 193-196]. However, many of the abovementioned and other theories/hypotheses floating around for some time are being refuted or challenged.

Anti migraine therapy essentially consists of acute/abortive and prophylactic components.

In the recent past, several novel approaches to the treatment and prevention of migraine have been advanced. Ever since the successful introduction of ergotamine tartarate and dihydroergotamine, [Practice parameter: Appropriate Use of Ergotamine Tartarate and Dihydroergotamine in the Treatment of Migraine and Status Migrainosus (Summary Statement) : Report of the Quality Standards Sub-Committee of American Academy of Neurology, Neurology, March 1995, 45(3 Pt 1), 585-587] a wide array of drugs are available today to treat and prevent migraine.

The last decade has witnessed a tremendous progress in acute abortive therapy of migraine using a new class of drugs, viz. the "triptans", which are prototypes of the Serotonin 5-HT₁ agonists (Peroutka, S Developments in 5-hydroxytryptamine receptor pharamacology in migraine. Neurol. Clin. 1990, 8:829-839). The "triptans", primarily acting via 5-HT_{IB/D} receptor mechanism, can be administrated, nasally and orally and are found to be quick in action and generally provide 70% relief to migraine attacks in one hour compared to placebo (less than 27 %). However, some of the "triptans" exhibit certain pharmacodynamic and pharmacokinetic disadvantages, which limit their use for effective pharmacotherapy of migraine.

The number of agents for prophylactic treatment of migraine compared to that available for the abortive treatment are not large. The existing agents for the prophylactic therapy include, but are not limited to :
i) β-blockers, such as propranolol, metaprolol, nadolol, atenolol, and timolol which are effective in decreasing the frequency of attack [Stensrud, P. and Sjaastad O., Comparative Trial of Tenormin (atenolol) and Inderal (propranolol) in Migraine, Headache, July 1980, 20 (4), 204 ; Kangasniemi et. al., Classic Migraine: Effective Prophylaxis with Metoprolol; Cephalagia, 1987, suppl 6, 464 ; Diamond, S. and Medina J.L., Double Blind Study of Propranolol in the Prophylaxis, Headache, March 1976, 16(1), 24-27; Nadelmann, J.W. et. al., Propranolol in the Prophlaxis of Migraine, Headache, April 1986, 26(4), 175-182].
   However, it is not clear whether their role in achieving prophylaxis is through catecholaminergic system or through 5-HT₂ receptors.
ii) Calcium ion channel antagonists, such as flunarizine and verapamil, which bring about a reduction in the frequency of attack [Welch, K. et. al., Central Neurogenic Mechanisms of Migraine, Neurology, 1993, 43 (suppl), S21-25].
iii) Serotonin 5-HT₂ receptor antagonists such as methysergide and pizotyline. The former is particularly effective in cases where the attack is severe, have high recurrence and do not respond to other medication [Welch, K. et. al., Central Neurogenic Mechanisms of Migraine, Neurology, 1993, 43 (suppl), S21-25].
iv) Tricyclic antidepressants, like amytryptiline and notryptiline given when the attack is aggravated by tension, depression and insomnia [Couch, J. and Hassanein, R.S., Amitriptyline in Migraine Prophylaxis, Arch. Neurol., 1979, 36, 695-699].
v) Monoamino oxidase inhibitors, like phenelzine and isocarboxazid, given in cases where the headaches are refractory to standard treatment. These drugs are believed to have the ability to increase the levels of endogenous 5-HT and thereby useful in migraine prophylaxis [Peatfield, R.C. et. al., Drug Treatment of Migraine Handbook of Clinical Neurology: (Rose F.C ed.), Raven Press, New York, 1986, 4, 173-216].
vi) Anti-epileptic drugs such as sodium valproate, valproic acid and divalprox, effective in cases where the migraine attacks are associated with seizures, mania or anxiety [Jensen, R. et. al., Sodium Valproate has a Prophylactic Effect in Migraine without Aura: A Triple Blind, Placebo Controlled Crossover Study, Neurology, April 1994, 44 (4), 647-51; Mathew, N.T. et. al., Migraine Prophylaxis with Divalpro, Arch. Neurology, 1995, 52, 281].

However, in addition to several side-effects and shortcomings such as constipation, rebound headache, lethargy, depression, impotence, loss of hair, nausea, muscle cramps, aching, claudication, weight gain, hallucinations, idiosyncratic retroperitioneal fibrosis, drowsiness, drying of mouth, blurred vision, urinary retension, cardiac arrhythmia, orthostatic hypotension, hepatotoxicity, alopecia, tremor etc. the rationale for administration and use of the abovementioned drugs is still not very clear.

The abovementioned shortcomings and non-availability of selective therapeutic agents have led to the search for newer effective anti-migraine agents for the prophylactic and abortive therapy with less side effects and less toxicity profile.

New targets are being investigated for the prophylactic therapy of migraine and epilepsy, which share several clinical features and in many instances, respond to the same pharmacological agent. This suggests that similar mechanism(s) may be involved in their respective pathophysiology [Cutrer, F.M, Antiepileptic Drugs: How they Work in Headache, Headache, 2001, (suppl) 1, s3-s10].

Amongst these, anticonvulsants as a class of drugs hold promise for migraine prophylaxis. These drugs are thought to act thorough multiple mechanisms involving voltage gated ion channels, ligand gated ion channels, GABA (y-Amino Butyric Acid), Glutamate, Glycine, combined voltage/ligand gated ion channels and NMDA (N-Methyl D-Aspartate) [Cutrer. F.M, Antiepileptic Drugs: How they Work in Headache, Headache, 2001, (suppl) 1, s3-s10].

In the central nervous system, GABA is a major inhibitory neurotransmitter and known anticonvulsant drugs like sodium valproate and gabapentine have been shown to be effective in preventing migraine through modulation of GABA neurotransmission [Hering, R. and Kinitzky, A., Sodium Valproate in the Prophylactic Treatment of Migraine : A Double Blind Study v/s Placebo, Cephalgia, 1992, 12(2), 81-84**;** Cutrer, F.M. et. al., Possible Mechanism of Valproate in Migraine Prophylaxis; Cephalagia, 1997, 17(2), 93-100; Magnus, L., Non Epileptic use of Gabapentine, Epilepsia, 1999, 40 (suppl 6), S66-S72].

Others like Carbamazepine, used for treatment of trigeminal neuralgia has also been shown to be effective in the prophylaxis of migraine, primarily mediated by sodium channels [Rompel, H. and Bauermeister, P.W., Aetiology of Migraine and Prevention with Carbamazepine (Tegretol): Results of Double Cross Over Study, S. Afri. Med J., 1970; 44, 75-78]. Lamotrigine, a glutamate antagonist that blocks voltage gated sodium channels has also been demonstrated to be effective in migraine prophylaxis with aura [Lampl, C. et. al., Lamotrigine in the Prophylactic treatment of Migraine-Aura : A Pilot Study, Cephalalgia, 1999, 19(1), 58-63]. Further, topiramate whose mechanism of action includes inhibition of voltage dependent sodium and calcium channels, AMPA (α-Amino-3-Hydroxy-5-Methyl-4-Isoxazolepropionic Acid)/Kainate glutamate receptors as well as enhancement of GABA-A receptor action is under extensive investigation as a prophylactic agent for migraine [Cutrer, F.M., Antiepileptic Drugs: How they Work in Headache, Headache, 2001, (suppl) 1, s3-s10].

Anticonvulsant, topiramate is highly effective in maximal electroshock seizure test in rats and mice [Shank, R.P. et.al., Topiramate: Preclinical Evaluation of Structurally Novel Anticonvulsants, Epilepsia, 1994, 35(2), 450-460]. Efficacy of topiramate in migraine prophylaxis was recently documented [Von Seggern, R.L. et. al., Efficacy of Topiramate in Migraine Prophylaxis: A Retrospective Chart Analysis, Headache, 2002, 42 (8), 804-809]. The role of newer anticonvulsants topiramate and gabapentine are being evaluated in preventive migraine therapy and their rationale and use in treating neuropathic pain was recently reported [Corbo, J., The Role of Anticonvulsants in Preventive Migraine Therapy, Curr. Pain Headache Rep., 2003, 7(1), 63-66; Chong, M.S. and Libretto, S.E., The Rationale and Use of Topiramate for Treating Neuropathic Pain. Clin. J. Pain 2003; 19(1) 59-68].

It might be mentioned here that all the anticonvulsants tested/under testing for the prophylaxis of migraine involve administration of the drug through routes other than nasal and their mechanism of action is not very clear.

Nasal sprays or drops are known for quick relief of migraine headaches For example, nasal sprays/drops containing dihydroergotamine, sumatriptan succinate and lidocaine have been reported and used commercially [Selby, G. and Lance, J.W., Observations on 500 Cases of Migraine and Allied Vascular Headache, J. Neurol. Neurosurg. Pschiatry, 1960, 23, 23-32; Boureau, F. et. al., A Clinical Comparison of Sumatriptan Nasal Spray and Dihydroergotamine Nasal Spray in the Acute Treatment of Migraine, Int. J. Clin. Pract., 2000, 54, 281-286; Maizels, M. et. al., Intranasal Lidocane for Treatment of Migraine: A Randomized Double Blind Controlled Trial, JAMA, 1996, 276(40), 319-321; Diamond, S., A Fresh Look at Migraine Therapy, Post Grad. Med., 2001, 109(1), 49-60].

A possible treatment of and relief from migraine has been reported through intranasal administration of an extract of *S.trifoliatus,* also known as Ritha or Arishta, which belongs to the family *Sapindaceae.* [Nadkarni, A.K., The Indian Materia Medica, Vol I, 2nd Edition, 1982, pp 1102-03, published by Bombay Popular Prakashan, Bombay, India]. The therapy generally practiced consists of preparing an aqueous solution of the extract of *S.trifoliatus* and administrating the same nasally.

However, there are no documented reports available which describe the concentration of the active ingredient, the dosage and duration of treatment and also it is not clear whether this mode of treatment is curative or prophylactic. In addition, the aqueous solution containing the extract of *S.trifoliatus* is generally prepared fresh, prior to administration, since the solution has no appreciable shelf life or stability. More importantly, Ritha is a potential irritant and thick pulp like solution is known to cause damage and severe irritation of the nasal mucusa, when administrated nasally. The above mentioned shortcomings severely limit the use of *S.trifoliatus* for treatment of migraine, unless improved.

PCT Application No. WO 01/89544 (D. B. Gupta et. al.) discloses a pharmaceutical composition containing a mixture of extracts of *S.trifoliatus* and *Emblica officinalis* in admixture with pharmaceutically acceptable additives having a pH of between 3.5 and 7.0, useful for the prophylactic treatment of migraine. The applicants have reported that the said composition, containing a mixture of extracts of *S.trifoliatus* and *Emblica officinalis* has a synergistic effect in the prophylactic treatment of migraine and moreover, is stable and does not cause damage or irritation to nasal mucous membrane, when administrated intranasally.

The abovementioned patent application also discloses a process for preparation of the said composition comprising the steps of:
a) soaking the pericarp of the fruit of *S.trifoliatus* and the dried fruit of *Emblica officinalis* in water for 1 to 8 days, preferably 7 days, in a closed container, with concomitant purging of the whole system with nitrogen gas during the entire soaking period.
b) filtration of the extract thus obtained after soaking.
c) addition of the pharmaceutically acceptable additives at any one of the following stages, viz. prior to the step of soaking and filtration; after the step of soaking and filtration; after the soaking step but prior to filtration.
d) adjusting the pH of the solution in the range of between 3.5 to 7.0.
e) making up of the solution to the desired concentration with water, and
f) finally storing the formulated solution in a bottle, which is purged with nitrogen gas before sealing.

The pharmaceutically acceptable additives used in the composition include,
i) an astringent e.g. aluminium potassium sulphate (alum),
ii) a suspending agent e.g. xanthan gum, guar gum, hydroxypropyl methyl cellulose, hydroxypropyl cellulose etc.,
iii) an isotonic agent e.g. sodium chloride,
iv) a preservative e.g. benzalkonium chloride, chlorbutanol, sodium methyl paraben, sodium propyl paraben and phenethyl alcohol,
v) a sequestering agent e.g. disodium EDTA,
vi) an antioxidant e.g. sodium meta bisulphite, and
vii) a pH adjusting agent e.g. sodium hydroxide, sodium phosphate, sodium citrate, sodium carbonate, sodium ascorbate etc.

However, the composition mentioned hereinabove is associated with the following shortcomings, viz.
a) involves the utilization of two active principals, viz. *S.trifoliatus* and *Emblica officinalis*
b) involves a lengthy extraction and soaking process of the active principals taking at least 7 days
c) use of nitrogen gas throughout the period of soaking and extraction
d) use of a number of pharmaceutically acceptable additives, and in particular
e) use of alum, which is a known irritant and a corrosive chemical in the composition
all of which taken in conjunction not only lead to increase in the cost and time of manufacture but also renders the composition less safe.

There exists a need, therefore, for a method of treatment of migraine, which addresses the shortcomings of the existing methods and which, moreover, is safe, less expensive and is convenient, which forms the objective of the present invention.

### OBJECTS OF THE INVENTION

It is therefore, an object of the present invention to provide a pharmaceutical composition for treatment of various disorders related to the binding affinities for the receptor sites viz. GABA-A agonist site, Glutamate-AMPA site, Glutamate-Kainate site, Glutamate-NMDA agonistic site, Glutamate-NMDA glycine (strychnine insensitive) site and Sodium channel (site 2), which are known to have major mediatory role in anticonvulsant activity.

Another object of present invention is to provide a pharmaceutical composition for treatment of migraine, which is safe, well tolerated, and non-toxic, with minimal and reversible adverse reactions or side effects.

A further object of the present invention is to provide a process for preparation of the composition, which is selective, simple, efficient and cost-effective.

### SUMMARY OF THE INVENTION

In their endeavour for identification and characterization of new prophylactic targets for migraine the present inventors have found that an herbal extract, containing a mixture of triterpenoid saponins derived from *S.trifoliatus* exhibits excellent anticonvulsant activity. The anticonvulsant activity exhibited by the extract in particular, is found to be highly suitable for the prophylactic treatment of migraine.

In addition, the extract was found to exhibit receptor binding affinity towards GABA-A agonist site, Glutamate-AMPA site, Glutamate-Kainate site, Glutamate-NMDA agonistic site, Glutamate-NMDA glycine (strychnine insensitive) site and Sodium channel (site 2), which apart from being novel and hitherto not known, provide a highly efficient, convenient, safe and cheap method for the prophylactic treatment of migraine.

Further, the present inventors have found that the herbal extract containing a mixture of triterpenoid saponins derived from *S.trifoliatus,* could be prepared by a process, wherein the active principals, viz. a mixture of saponins could be extracted from the pericarp of the fruit of *S.trifoliatus,* using water or an alcohol or a mixture thereof in a short duration of time of between 0.5 to 24 hours and more, importantly in the absence of an inert gas atmosphere such as nitrogen.

In addition, the present inventors have found that the aqueous, alcoholic or a hydroalcoholic extract containing a mixture of triterpenoid saponins derived from *S.trifoliatus,* thus obtained could be formulated into a pharmaceutical composition with utilization of lesser number of pharmaceutically acceptable additives as compared to the composition of the prior art.

Further, the present inventors have found that the aqueous, alcoholic or a hydroalcoholic extract containing a mixture of triterpenoid saponins derived from *S.trifoliatus* could be constituted into a pharmaceutical composition for administration through the nasal route, without utilization of an astringent like alum in the composition.

Finally, the present inventors have found that the aqueous, alcoholic or hydroalcoholic extract containing a mixture of triterpenoid saponins derived from *S.trifoliatus* are estimated to contain 4-8 (%w/w) of hederagenin. The extract does not cause damage or irritation to the nasal mucous membrane, when administrated intranasally, thereby providing a safe, simple, convenient and cost-effective method for treatment of migraine.

In summary, the present invention provides a pharmaceutical composition containing an extract comprising a mixture of triterpenoid saponins derived from *S.trifoliatus,* which further comprises 0.001 to 1.0 (%w/v), of hederagenin, which exhibits excellent anticonvulsant activity, which in turn when administrated intranasally is suitable for the prophylactic treatment of migraine. The invention also provides a simple and convenient process for preparation of the said extract, which
i) obviates the use *of Emblica officinalis* in order to achieve a synergistic effect
ii) obviates the need to use the alum as astringent,
iii) utilizes lesser number of additives,
iv) does not cause damage or irritation to the nasal mucous membrane, and
v) can be prepared in a simple manner in a shorter duration of time and does not take recourse to inert gas atmospheric conditions,

which collectively offer advantages on a commercial scale and thereby providing a safe, simple, convenient and cost-effective method for treatment of migraine.

Thus in accordance with the abovementioned :
In one aspect of the present invention, there is provided an aqueous, alcoholic or a hydroalcoholic extract, containing a mixture of triterpenoid saponins derived from the pericarp of fruits of the plant species *S.trifoliatus,* possessing useful pharmacological activity.
In another aspect of the present invention, there is provided an aqueous, alcoholic or a hydroalcoholic extract, containing a mixture of triterpenoid saponins derived from the pericarp of fruits of the plant species *S.trifoliatus,* possessing anticonvulsant activity.
In yet another aspect of the present invention, there is provided an aqueous, alcoholic or a hydroalcoholic extract, containing a mixture of triterpenoid saponins derived from the pericarp of fruits of the plant species *S.trifoliatus* useful for the prophylactic treatment of migraine.
In a further aspect of the present invention, there is provided an aqueous, alcoholic or a hydroalcoholic extract, containing a mixture of triterpenoid saponins derived from the pericarp of fruits of the plant species *S.trifoliatus* for the prophylactic treatment of migraine, mediated through its anticonvulsant activity.
In another further aspect of the present invention, there is provided an aqueous, alcoholic or a hydroalcoholic extract, containing a mixture of triterpenoid saponins derived from pericarp of *S.trifoliatus* wherein the said extract is highly effective for human use and capable for being used for the prophylactic treatment, relief and remedy of migraine.
In yet another further aspect of the present invention, there is provided a process for the preparation of the aqueous, alcoholic or a hydroalcoholic extract, containing a mixture of triterpenoid saponins from the pericarp of the fruit *of S.trifoliatus.*
In yet another aspect of the present invention, there is provided a process for the preparation of pure compounds from a mixture of triterpenoid saponins from the pericarp of *S. trifoliatus.*

Another aspect of the present invention is evaluation of the aqueous, alcoholic or a hydroalcoholic extract, containing a mixture of triterpenoid saponins derived from *S.trifoliatus* for its *in vitro* receptor binding affinity towards the selected receptors, which have mediatory role in anticonvulsant activity.

Another aspect of the present invention is to provide a pharmaceutical composition containing a pharmaceutically effective amount of an extract, containing a mixture of triterpenoid saponins derived from *S.trifoliatus* useful in the treatment of certain indications.

A final aspect of the present invention is to provide a pharmaceutical composition containing a pharmaceutically effective amount of an extract, containing a mixture of triterpenoid saponins derived from *S.trifoliatus* useful in the prophylactic treatment of migraine.

### DESCRIPTION OF THE ABBREVATIONS/NOTATIONS

The following abbrevations/notations used throughout the text refer to the following:
**[1]** Pericarp of S. *trifoliatus*
**[2]** Extract of the pericarp of *S.trifoliatus* containing a mixture of saponins
**[3]** Dry powder obtained on lyophilization of the aqueous extract of the pericarp of *S.trifoliatus*
**[4]** Pharmaceutical Composition containing lyophilized aqueous extract [3] of the pericarp of *Sapindus trifoliatus* in admixture with pharmaceutically acceptable additives.
**[5-10]** Pure compounds are the pure saponins (hedragenin derivatives) isolated from the extract of the pericarp of *S.trifoliatus.*

### DETAILED DESCRIPTION OF THE INVENTION

*S.trifoliatus,* known as Ritha or Aristha belongs to the family of *Sapindaceae.* The fruit of the plant is used therapeutically as a tonic, purgative, emetic and expectorant [Nadkarni, A.K., The Indian Materia Medica, Vol I, 2nd Edition, 1982, pp 1102-03, published by Bombay Popular Prakashan, Bombay, India]. It also possesses antiinflammatory and analgesic actions. It is also used as a spermicidal, in treatment of piles, hysteria, epilepsy and anti-implantation [Pharmaceutical Investigations of Certain Medicinal Plants and Compound Formulations used in Ayurveda and Siddha, Published by CCRAS, New Delhi, India, 1996, pp 22-25].

The pericarp of the fruit of the plant, which constitutes 62% of the fruit contains, glucose, saponins and primary metabolites. The saponins present in the fruit on acidic hydrolysis give the triterpenoid hederagenin, D-glucose, L-rhamnose and D-xylose and Arabinose. [The Wealth of India, Vol IX, CSIR Publication, by NISCOM, New Delhi, India, 1998, pp 227-29].

*S.trifoliatus* is pungent and bitter in taste. It has emetic actions i.e. it causes vomiting and nausea and is known to cause irritation of gastric mucosa, when administrated orally (Sharma, Dravyagunavignan, VIII Ed., 1986, pp 384-86.)

As mentioned hereinearlier, administration of *S.trifoliatus* through nasal route is indicated for treatment of hemicrania [Nadkarni, M.K., The Indian Materia Medica, Vol I, 2nd Edition, 1982, pp 1102-03, published by Bombay Popular Prakashan, Bombay, India]. The therapy generally practiced consists of preparing an aqueous solution of *S.trifoliatus* and administration of the same through nasal route. However, there is no suggestion from the prior art about the effective concentration of the active ingredient, the preferred dosage required and duration of treatment. Moreover, it is not clear whether it is used as a curative or prophylactic and most importantly, its mechanism of action. In addition, the formulated solution needs to be prepared fresh all the time, as it has no appreciable shelf life or stability as mentioned in prior art.

The pericarp of the fruit *of S.trifoliatus* is utilized for preparation of the pharmaceutical composition **[4]** as per the present invention, wherein the active ingredient i. e. pericarp of the fruit of *S.trifoliatus* **[1]** can be used either in the coarse form as such or it can be pulverized before use

The pericarp of the fruit of *S.trifoliatus* **[1]** can be extracted by percolation with water or an alcohol or mixtures thereof at ambient temperatures for a period ranging from 0.5 to 20 hours, preferably 14-16 hours. Alternatively, the pericarp can be extracted by boiling it with water or an alcohol or mixtures thereof for 4-5 hours.

Suitable alcohols can be selected from those having C₁₋₆carbon atoms, both straight and branched. Preferred alcohols are ethanol, n-propanol, iso-propanol, n-butanol, iso-butanol and tert-butanol. The ratio of water to alcohol when a mixture is used is not important and can be of individual choice.

The extracts obtained from all the three modes of extractions i.e. aqueous, alcoholic and hydroalcoholic extracts **[2]** show the presence of the principal saponins and other primary metabolites as evidenced by TLC and HPLC.

The saponins present in the aqueous, alcoholic or aqueous-alcoholic extract **[2]** have been isolated and identified. The aqueous, alcoholic or aqueous-alcoholic extract of S. *trifoliatus* **[2]** was fractionated with n-butanol. The butanol layer was concentrated to give a solid. This was dissolved in methanol and adsorbed on silica gel. The column was eluted with chloroform-methanol with increasing proportions of methanol (2, 4, 6 etc.). Fractions were collected to yield six crude compounds. Further purification was done by repeated flash chromatography on silica gel to yield compounds **5-10,** again using chloroform-methanol with increasing proportions of methanol (2, 4, 6 etc.). Each of these 6 hedragenins derivatives were characterized and identified by spectral methods.
- Compound **[5]**: Hederagenin-3-O-(□-L-arabinopyranosyl-(1---3)-□L-rhamnopyranosyl-(1---2)-□-D-xylopyranoside
- Compound **[6]**: Hederagenin-3-O-(3-O-acetyl-□-D-xylopyranosyl-(1---3)-□-L-rhamnopyranosyl-(1---2)-□-L-arabinopyranoside
- Compound **[7]**: Hederagenin-3-O-(4-O-acetyl-□-D-xylopyranosyl-(1---3)-□-L-rhamnopyranosyl-(1---2)-□-L-arabinopyranoside
- Compound **[8]**: Hederagenin-3-O-(3,4-O-diacetyl-□-D-xylopyranosyl-(1---3)-□-L-rhamnopyranosyl-(1---2)-□-L-arabinopyranoside

- Compound **[9]**: Hederagenin -3-O-(□-D-xylopyranosyl-(1---3)-□L-rhamnopyranosyl-(1---2)-□-D-xylopyranoside
- Compound **[10]**: Hederagenin-3-O-(□-D-xylopyranosyl-(1---3)-□L-rhamnopyranosyl-(1---2)-□-D-xylopyranoside

Acid hydrolysis of the extract yielded only one aglycone, which was identified as hederagenin. Therefore, estimation of the abovementioned saponins present in the aqueous, alcoholic or aqueous-alcoholic extract **[2]** was calculated as hederagenin. The content of hederagenin was estimated in the extract by boiling it with 50% methanolic HCl. The entire mixture was evaporated to dryness. This was reconstituted in methanol and estimated by HPLC. The concentration of hederagenin was found to be between 4-8 % w/w of the extract.

The saponins present in the aqueous, alcoholic or aqueous-alcoholic extract **[2]** have stability of 9 months.

The aforementioned herbal extract exhibits receptor binding affinity towards GABA-A agonist site, Glutamate-AMPA site, Glutamate-Kainate site, Glutamate-NMDA agonistic site, Glutamate-NMDA glycine (strychnine insensitive) site and Sodium channel (site 2), As mentioned herein earlier, the receptor binding affinity exhibited by the aforesaid extract is new and hitherto not known and which constitutes an important aspect of this invention.

The extract **[2]** is useful in treatment of certain indications, such as hysteria, epilepsy, pain, asthma etc, in particular the prophylactic treatment of migraine. The receptor binding activity exhibited by the extract **[3]** is useful in anticonvulsant activity. This anticonvulsant activity is believed to be useful in the prophylactic treatment of migraine.

*in vitro* receptor binding studies reveal that the extract of *S.trifoliatus* **[3]** exhibits binding affinity towards the receptor sites, which have a major mediatory role in its anticonvulsant activity

The selected receptor binding affinity studies with the extract of *S.trifoliatus* **[3]** were conducted at NOVASCREEN®, USA for GABA_{A} agonist site, Glutamate-AMPA site, Glutamate-Kainate site, Glutamate-NMDA agonistic site, Glutamate-NMDA glycine (strychnine insensitive) site and Sodium channel (site 2).

The results obtained on the above studies using the extract of *S.trifoliatus* **[3]** is summarized below in Table-I.

The extract **[2]** can be used as such or preferably is lyophilized **[3]** and the lyophilized material thus obtained is reconstituted with appropriate quantity of water to achieve the desired concentration before use. Similarly, from the alcohol extract the solvent is evaporated to dryness under reduced pressure and further reconstituted with appropriate quantity of water to achieve the desired concentration before use. The hydroalcoholic extract can be initially evaporated under reduced pressure and then lyophilized and further reconstituted with water.

**Table-I : Receptor binding affinity studies with the extract of S.trifoliatus [3]**

| Receptor | Receptor Source | Ligand | % Inhibition | |
|---|---|---|---|---|
| | | | 2.5 µg/mL * | 250 µg/mL * |
| GABA A, Agonistic site | Bovine Cerebellum | [³H]GABA | 50.92 | 102.40 |
| Glutamate, AMPA site | Rat Forebrain | [³H]AMPA | 5.43 | 87.36 |
| Glutamate, Kainate site | Rat Forebrain | [³H]Kainic acid | -15.70 | 87.29 |
| Glutamate, NMDA agonist site | Rat Forebrain | [³H]CGP 39653 | 7.27 | 98.14 |
| Glutamate, NMDA, Glycine (Strychnine-insensitive site) | Rat Cortex + Hippocampus | [³H]-NML-105,519 | 14.50 | 85.33 |
| GABA, Chloride, TBOB | Rat Cortex | [³H]TBOB | -5.12 | 85.03 |
| Glutamate, Chloride | Rat Cerebellum | [³H]Glutamic Acid | -2.72 | 89.49 |
| Sodium, Site 2 | Rat Forebrain | [³H] Batrachotoxin A 20-a-Benzo | 19.98 | 69.54 |

| | | | | |
|---|---|---|---|---|
| (*) Refers to the lyophilized powder obtained from the aqueous extract *of Sapindus trifoliatus* | | | | |

Further, the extract of *S.trifoliatus* **[3]** exhibited dose dependent binding affinity to GABA_{A} agonistic site, with IC₅₀ value of 1.74 µg/ml (Ki 1.70 µg/ml). The extract of *Sapindus trifoliatus* **[3]** also exhibited dose dependent binding affinity to glutamate-NMDA agonistic site with IC₅₀ of 140 µg/ml (Ki 113µg/ml).

The IC₅₀/Ki determination study for GABA_{A} agonist site and Glutamate-NMDA indicate that the extract has dose dependent binding affinity to GABA_{A} agonistic site and glutamate-NMDA agonistic site.

From *in vivo* studies it is observed that the extract **[3]** prevented the hind limb extensor phase in rats, which moreover, is dose dependent in a Maximal Electroshock Seizure (MES) model. This clearly indicates prevention of seizure spread on intranasal administration.

### Irritancy studies

Intranasal medication up to 3%w/v of the active ingredient **[1]** in rats and 1% w/v of the active ingredient **[1]** in dogs for 28 days and was non-irritant to nasal tract, turbinate bones bronchi and lungs. No effect of the medication was observed on other organs of the animal.

### Description of the Method of Evaluation for Anticonvulsant Activity in MES Model

The nature of the binding affinity of the extract of *S.trifoliatus* was further investigated in functional assays using *in vivo* animal models.

In order to evaluate the efficacy of the extract of *Sapindus trifoiliatus* **[3],** for its prophylactic therapeutic potential in migraine, its role as an anticonvulsant was evaluated in an *in vivo* animal model. Maximal Electroshock Seizure (MES) ([Swinyard, E et. al., Comparitive Assays of Antiepileptic Drugs in Mice and Rats, J. Pharmacol. Exp. Ther., 1952, 106, 319-330] test model was employed for the efficacy evaluation. Drugs acting on the receptors like Glutamate-NMDA,Glutamate-AMPA /Kainate,Glycine site and voltage dependent Na⁺ channels are known to inhibit MES induced seizures [Lin, S.S. and Sun, L.R., A Novel Anticonvulsant with a Dual Mechanism of Action, CNS Drug Reviews, 1999, 5(4), 365-378; White, H.S. et. al., The Early Identification of Anticonvulsant Activity : Role of maximal Electroshock and Subcutaneous Pentylenetetrazole Seizure Models, Ital. J. Neurol. Sci., 1995, 16(1-2), 73-77].

Male Wistar rats (150 - 200 g) were used in the study. The extract **[3]** dissolved in saline was administered intranasally in a volume of 250µl/kg in a dose range of 0.25mg/kg to 25mg/kg. After administration of either the test compound or an equivalent volume of the vehicle (for control experiments) or standard drug, the rats were observed for any tremors or convulsions. Thirty minutes after intranasal administration the rats were administered electroshock (100Hz, 150mA, 0.2 sec) by bipolar pinna electrodes using an electroconvulsometer (INCO, India). The incidence, latency as well as duration of hind limb extension were noted. Mortality if any was recorded. Abolition of the hind limb tonic extensor component indicates the test compound's ability to inhibit MES-induced seizure spread.

Values of incidence and mortality were expressed as ratios and analysed by Fisher's test. The latency for onset and duration of hind limb extension of maximal electroshock induced convulsions were averaged, expressed as mean ± standard deviation. Mean values were analysed by one way ANOVA followed by Dunnett 't' test for multiple comparison or Students 't' test for comparing two means. A p<0.05 was considered statistically significant. Statistical analysis was done using the Graph Pad^{®} software, USA. ED₅₀ values were calculated by probit analysis [Finney, D.J., Probit Analysis, Cambridge University Press, London, 1947**].**

The extract **[3]** administered intranasally at a dose range of 2.5mg/kg to 25mg/kg in a volume of 250µl/kg abolished the hind limb tonic extensor phase in the MES induced seizures in rats. The ED₅₀ for the extract of *Sapindus trifoiliatus* was determined to be 7.72 mg/kg, i.n., while that of Sodium valproate was 67.70 mg/kg, i.p., as summarized below. ED₅₀ represents protection to hind limb tonic extension due to electroshock and the same is summarized in Table-II.

**Table-II : The ED₅₀ values for the extract of Sapindus trifoiliatus [3] in comparison with that of Sodium Valproate**

| **Treatment*** | **ED₅₀ values** | **95% confidence limits** |
|---|---|---|
| ***S.trifoliatus*** extract **[3]** | 7.72 mg/kg, i.n. | 5.28 to 11.04 mg/kg i.n. |
| ***Sodium valproate*** | 67.67 mg/kg, i.p. | 53.53 to 80.30 mg/kg i.p. |

| | | |
|---|---|---|
| (*) No. of animals at each treatment level 5-10. | | |

The extract *of S.trifoliatus* **[3]** did not cause protection against PTZ induced convulsions in rats on intra-nasal administration.

### Description of the Method of Evaluation for Anticonvulsant Activity in PTZ Model

Pentylenetetrazole (PTZ) seizure test [Snead, O.C., Pharmacological Models of Generalized Absence Seizures in Rodents, J. Neurol. Transm., 1992, (suppl) 35, 7-19]. model was employed for the efficacy evaluation. Male Wistar rats (150 - 200 g) were used in the study. The extract of *Sapindus trifoiliatus* **[3]** dissolved in saline was administered intranasally at two high concentrations 250 mg/kg and 375 mg/kg in a volume of 250µl/kg based on solubility and syringability for instillation into the nasal cavity. After administration of either the test compound or an equivalent volume of the vehicle (for control experiments) or standard drug, the rats were observed for any tremors or convulsions. Fifteen minutes after intranasal administration rats were administered pentylenetetrazole (60 mg/kg,i.p. 2 ml/kg) and the incidence and latency of myoclonic jerks as well as generalized seizures were noted for a period of 30 minutes. Also severity was ranked on a scale of 0-5. Mortality, if any were recorded. Severity was ranked as follows:
Stage 0 -No response,
Stage 1 - Ear and facial twitching,
Stage 2- Myoclonic jerks without upright posture,
Stage 3 - Myoclonic jerks, upright position with bilateral forelimb clonus,
Stage 4- Clonic tonic seizures, and
Stage 5 - Generalized clonic - tonic seizures, loss of postural control.
   Diazepam (4mg/kg,i.p., 2ml/kg) was used as the standard control. Absence of generalized clonic convulsions of stage 5 severities indicates compound's ability to be protective in nature.

### Statistical Analysis of Data

Values of incidence and mortality were expressed as ratios and analysed by Fisher's test.
The latency for onset of myoclonic jerks and generalized clonic seizures were averaged, expressed as mean ± standard deviation. Mean values were analysed by one way ANOVA followed by Dunnett 't' test for multiple comparison or Students 't' test for comparing two means. Severity rankings were averaged and expressed as mean ± standard deviation and the medians were analysed by Kruskal-Wallis non-parametric test followed by Dunn's test to compare sum of ranks. A p<0.05 was considered statistically significant. All statistical analysis were done with Graph Pad^{®} (U.S.A.) software. ED₅₀ values were calculated by probit analysis [Finney, D.J., Probit Analysis, Cambridge University Press, London, 1947].

The extract of *S.trifoliatus* **[3]** administered intranasally at two high concentrations 250mg/kg and 375 mg/kg in a volume of 250µl/kg did not afford protection to PTZ induced seizures in rats. However diazepam (4mg/kg,i.p., 2ml/kg) used as the standard control significantly protected the seizures induced due to PTZ. The rats did not show any tremors or convulsions due to *S.trifoliatus* treatment prior to PTZ administration.

The extract of *S.trifoliatus* **[3]** did not effect motor co-ordination in rats on intra nasal administration indicating lack of neurological impairment at the doses studied.

### Description of the Method of Evaluation of Motor Co-ordination on Rota Rod Performance Tests in Rats

Drugs with anticonvulsant activity that do not exhibit sedation or death in animal models are considered safe. Hence the effect of the extract of *S.trifoliatus* **[3]** was evaluated for the same on rota rod performance test in rats.

Wistar male rats (150 - 200g) pre-trained, were subjected to rotarod (Letica, Spain) test (15 rpm) for sixty seconds at intervals of 0, 5, 10, 15, 20, 30 and 45 minutes post intranasal treatment of test compound or an equivalent volume of the vehicle [Dunham, M.S. and Miya T.A., A Note on Simple Apparatus for Detecting Neurological Deficit in Rats and Mice, J. Amer. Pharmac. Assoc. Sci. Edit., 1957, 46, 208-209]. The extract of *S.trifoliatus* **[3]** dissolved in saline was administered intranasally at two high concentrations 250 mg/kg and 375 mg/kg in a volume of 250µl/kg based on solubility and syringability for instillation into the nasal cavity. The inability to balance for sixty seconds was considered as lack of motor in-coordination by the compound. Diazepam (4mg/kg,i.p., 2ml/kg) was used as the standard control. The number of animals passing the test were expressed as ratios and analysed by Fisher's test.

At the doses of 250 mg/kg and 375 mg/kg in a volume of 250µl/kg administered intranasally to rats , the extract of *S.trifoliatus* **[3]** did not affect motor co-ordination upto 45 minutes post treatment in rotarod performance test. There were no noticeable tremors or convulsions in *S.trifoliatus* treated rats as compared to the control group. Drugs with anticonvulsant activity that do not exhibit sedation or death in animal models are considered safe.

Further studies suggest that the extract **[3],** which shows affinity towards receptors that have a mediatory role in anticonvulsant activity, however, does not induce or potentiate convulsions of chemical or electrical origin.

Preclinical pharmacological data from receptor binding and *in vivo* studies clearly indicate anticonvulsant activity of the extract **[3].** The anticonvulsant activity has been demonstrated in the MES model by the intra nasal route of administration without sedation.

The toxicological studies for acute lethality dose (LD₅₀) of the extract of *S.trifoliatus* **[3]** were conducted in both mice and rat by using intra nasal route. Further, to find out lethal dose by other routes (both intravenous and oral) were also employed. Mice and rats were observed for a period of 14 days after treatment with the extract.

The acute lethality dose (LD₅₀,mg/kg) of the extract of *S.trifoliatus[3]* was found to be >270 (intranasal), >1250 (oral) and >150 (intravenous) in mice while in rats it was found to be >90 (intranasal), > 1000 (oral) and >80 (intravenous).

The extract of *S.trifoliatus* **[3]** is further found to be safe in safety pharmacological studies. The study includes central nervous system, cardiovascular, gastrointestinal, urinary systems as well as spasmogenic, anti-aggregatory and haemolytic effects.

Active ingredient *S.trifoliatus* **[1],** at a maximum strength of 3% equivalent to 9.05mg/ml of aqueous extract 10ml/kg oral and 1 ml/kg intranasal did not exhibit any significant effect in mice on pentobarbitone induced sleeping time, locomotor activity, electroshock & PTZ induced seizures, acetic acid induced writhing and in the Irwin battery. Also, the extract **[3]** at 10 ml/kg oral and 0.25ml/kg intranasal did not exhibit any significant effect in rats on, motor co-ordination and analgesic activity by tail flick method.

Active ingredient *S.trifoliatus* **[1]** at a maximum strength of 3% equivalent to 9.05mg/ml of aqueous extract, 10ml/kg oral and 0.25ml/kg intranasal exhibited no significant effect in rats on cardiovascular system of conscious freely moving rats (on blood pressure and heart rate), gastro-intestinal system, urinary system and autonomic nervous system.

In *in-vitro* studies the aqueous extract of *S.trifoliatus* **[3],** did not show any haemolysis upto 100µg/ml in rat, rabbit and human blood.

In *in-vitro* smooth muscle contractility studies on guinea pig ileum, the aqueous extract of *S.trifoliatus* **[3]** did not show any spasmogenic or anti-muscarinic activity up to 30µg/ml.

The aqueous extract of *S.trifoliatus* **[3]** did not show any *in-vitro* platelet anti-aggregatory effect upto 1000 µg/ml.

Batches of nasal spray **[4]** containing the lyophilized aqueous extract of *S.trifoliatus* **[3]** equivalent to 0.004, 0.013, 0.027 and 0.08 (%w/v) of hederagenin have been formulated in combination with suitable pharmaceutically acceptable carriers or vehicles (Table-III).

For the process for preparation of the formulation 75% of batch volume of purified water was taken. Chlorobutanol was dissolved in ethanol and added to it under stirring. Phenylethyl alcohol was then added under stirring. After the solution became clear sodium chloride was added under stirring to the previous solution. Lyophilized aqueous extract of *S.trifoliatus* **[3]** equivalent to 0.004, 0.013, 0.027 and 0.08 (%w/v) of hederagenin, was then added and stirred to get a uniform dispersion. This dispersion was filtered through double fold nylon cloth. Xanthan gum was dissolved in 15% of batch volume of purified water under stirring. The previous dispersion was added to the solution of xanthan gum and stirred for 30minutes to homogenize the dispersion. The pH of the dispersion was checked and adjusted between 4.5-6.5 using 25%w/v solution of sodium citrate in purified water and stirred for 10 minutes. The final volume of the dispersion was then made up with purified water.

### Pharmaceutical Composition containing the extract of S.trifoliatus

The extract **[2]** can be administered as nasal drops, nasal sprays, nasal powders, semisolid nasal preparations, nasal washes and nasal sticks.

The composition may contain the extract of the pericarp of *S.trifoliatus* **[2]** in an amount where the range of hederagenin in composition is between 0.001-1.00 (%w/v), preferably 0.004 (%w/v) as nasal spray at a dose of 200 µl per day.

Suitable pharmaceutically acceptable carriers include sodium chloride to adjust tonicity; xanthan gum, carboxymethyl cellulose, methyl cellulose, hydroxy propyl methyl cellulose, polyvinyl pyrrolidone, polyvinyl alcohol, carbomers etc. to adjust viscosity; citric acid, sodium citrate, potassium dihydrogen phosphate, acetic acid, sodium acetate, ammonium acetate etc. to adjust pH and chlorbutanol , phenyl ethyl alcohol, parabens etc. as preservatives.

A preferred unit formula for nasal spray **[4]** containing the Extract of *S.trifoliatus* **[3]** for treatment of migraine is given in Table-III.

**Table-III : Unit formula for Nasal Spray [4] containing the Extract of S. trifoliatus**

| **Ingredient** | **%w/v** | | | |
|---|---|---|---|---|
| Strength in terms of %w/v Hederagenin | 0.004 | 0.013 | 0.027 | 0.08 |
| Aqueous extract **[3]** equivalent to hederagenin content | 0.004 | 0.013 | 0.027 | 0.08 |
| Chlorobutanol Hemihydrate BP | 0.40 | 0.40 | 0.40 | 0.40 |
| Ethanol (96%) BP | 0.40 | 0.40 | 0.40 | 0.40 |
| Phenylethyl Alcohol USP | 0.25 | 0.25 | 0.25 | 0.25 |
| Sodium Chloride I.P. | 0.90 | 0.90 | 0.90 | 0.90 |
| Xanthan Gum BP | 0.15-0.30 | 0.15-0.30 | 0.15-0.30 | 0.15-0.30 |
| Sodium Citrate I.P. or Citric Acid Monohydrate I.P. | q.s. to adjust pH 4.5-6.5 | q.s. to adjust pH 4.5-6.5 | q.s. to adjust pH 4.5-6.5 | q.s. to adjust pH 4.5-6.5 |
| Purified Water I.P. | q.s. | q.s. | q.s. | q.s. |

The product may be filled in suitable containers, capped with spray pump and actuator holding cap.

### Administration of the formulation [4] containing the extract of S.trifoliatus :

The formulation **[4]**containing the extract of S. *trifoliatus* prepared by the method described hereinabove can be administered intra-nasally two sprays, two times a day (2 X50 µl each) i.e. 200 µl per day for treatment of migraine.

The invention is further illustrated by the following examples.

### Example-1

### Extraction of the pericarp of S.trifoliatus with water

Dry pericarp of the fruit of Sapindus *trifoliatus* obtained from local suppliers was used as the starting material. 100 g of the pericarp was soaked in 400 ml of distilled water and left standing for 16 hrs. The percolate was then decanted, centrifuged and filtered through Whatman filter paper (No.1) to give a clear extract (300ml). The process of extraction was repeated three times with same volume of solvent. The percolate obtained in the second and third percolations were 400 ml each. These were pooled and lyophilized to give a brown coloured powder [3] in a yield of 68%.

### Example-2

### Extraction of the pericarp of S.trifoliatus with n-butanol

Dry pericarp of the fruit of *S.trifoliatus* (50.05g) was soaked in 250ml of n-butanol and left standing for 16 hrs. The percolate was then decanted, centrifuged and filtered through Whatman filter paper (No. 1) to give a clear extract (208ml). The process of extraction was repeated three times with same volume of solvent. The percolate obtained in the second and third percolations were 244 and 250 ml, each. These were pooled and lyophilized to give a brown coloured powder, in a yield of 13.51%

### Example-3

### Extraction of the pericarp of S.trifoliatus with iso-propanol

Dry pericarp of the fruit of *Sapindus trifoliatus* (50.06 g) was soaked in 250ml of isopropyl alcohol (IPA) and left standing for 16 hrs. The percolate was then decanted, centrifuged and filtered through Whatman filter paper (No. 1) to give a clear extract (205ml). The process of extraction was repeated three times with same volume of solvent. The percolate obtained in the second and third percolations were 240 and 246 ml, each. These were pooled and lyophilized to give a brown colored powder in a yield of 5.4%.

### Example-4

### Extraction of the pericarp of S.trifoliatus with aqueous ethanol

Dry pericarp of the fruit of *Sapindus trifoliatus* (25 g) was soaked in 100 ml of 50% aqueous-ethanol and left standing for 16 hrs. The percolate was then decanted, centrifuged to give a clear extract (86ml). This was lyophilized to give a brown coloured powder, in a yield of 55.0%.

### Example-5

### Isolation of saponins from pericarp of S.trifoliatus

Dry pericarp of the fruit of *Sapindus trifoliatus* (1 kg) was soaked in 5 Its of water and left standing for 16 hrs. The percolate was then decanted, centrifuged to give a clear extract (3.751). The process of extraction was repeated two more times with 3 Its of water each. The percolate obtained in the second and third percolations were 2.95 Its and 3.4 Its each. These were fractionated with n-butanol to give 255 g of solid. The solid was dissolved in 180 ml of methanol and adsorbed on 130 g of silica gel (60-120 mesh). The column was eluted with chloroform-methanol with increasing proportions of methanol (2, 4, 6 etc.). Fractions of 500 ml each were collected to yield compounds. Further purification was done by repeated flash chromatography on silica gel to yield compounds 5-10, which were further characterized and identified by spectral methods.

### Example -6

### Preparation of nasal spray [4] containing the lyophilized aqueous extract of S.trifoliatus [3] equivalent to 0.004 (%w/v) of hederagenin

750ml of purified water was taken. Chlorobutanol (4g) was dissolved in ethanol and added to it under stirring. Phenylethyl alcohol (2.5g) was then added under stirring. After the solution became clear sodium chloride (9.0g) was added under stirring to the previous solution. 1.51g lyophilized aqueous extract of *S.trifoliatus* **[3]** was then added and stirred to get a uniform dispersion. This dispersion was filtered through double fold nylon cloth. Xanthan gum (1.5g) was dissolved in 150ml of purified water under stirring. The previous dispersion was added to the solution of xanthan gum and stirred for 30minutes to homogenize the dispersion. The pH of the dispersion was checked and adjusted between 4.5-6.5 using 25%w/v solution of sodium citrate in purified water and stir for 10 minutes. The volume of the dispersion was then made up to 1 litre with purified water.

## Claims

1. Anticonvulsant pharmaceutical composition for nasal administration having binding affinities for the receptor sites viz. GABA-A agonist site, Glutamate-AMPA site, Glutamate-Kainate site, Glutamate-NMDA agonistic site, Glutamate-NMDA glycine (strychnine insensitive) site and Sodium channel (site 2), comprising
an aqueous, alcoholic or hydroalcoholic extract comprising a mixture of triterpenoid saponins derived from the pericarp of the fruit of Sapindus trifoliatus and pharmaceutically acceptable additives,
wherein the concentration of the triterpenoid saponins is calculated as hederagenin, said hederagenin amounting to 0.001 to 1 % w/v of the anticonvulsant pharmaceutical composition.

2. Anticonvulsant pharmaceutical composition according to claim 1, **characterised in that** the concentration of the triterpenoid saponins is calculated as a concentration of 0.004 to 0.08 % w/v hederagenin in the pharmaceutical composition.

3. Anticonvulsant pharmaceutical composition according to claim 1 or 2, **characterised in that** the extract is in the form of a lyophilized powder.

4. Anticonvulsant pharmaceutical composition according to any of claims 1 to 3, **characterised in that** the pharmaceutically acceptable additives comprise agents for adjusting the tonicity, viscosity, pH and a preservative agent.

5. Anticonvulsant pharmaceutical composition according to claim 4, **characterised in that** the agent for adjusting the tonicity is sodium chloride.

6. Anticonvulsant pharmaceutical composition according to claim 4 or 5, **characterised in that** the agent for adjusting the viscosity is selected from xanthan gum, carboxymethyl cellulose, methyl cellulose, hydroxypropyl methyl cellulose, polyvinyl pyrrolidone, polyvinyl alcohol and carbomers.

7. Anticonvulsant pharmaceutical composition according to any of claims 4 to 6,
**characterised in that** the agent for adjusting the pH is selected from citric acid, sodium citrate, potassium dihydrogen phosphate, acetic acid, sodium acetate and ammonium acetate.

8. Anticonvulsant pharmaceutical composition according to any of claims 4 to 7, **characterised in that** the preservative agent is selected from chlorbutanol, phenyl ethyl alcohol and parabens.

9. Anticonvulsant pharmaceutical composition according to any of claims 1 to 8, **characterised in that** the pH is in the range of 4.5 to 6.5.

10. Anticonvulsant pharmaceutical composition according to any of claims 1 to 9, **characterised in that** the composition is in the form selected from nasal drops, nasal sprays, nasal powders, semisolid nasal preparations, nasal washes or nasal sticks.

11. A process for the preparation of an extract containing a mixture of triterpenoid saponins, the process comprising the steps of
a) extraction of the pericarp of the fruit of Sapindus trifoliatus with water or an alcohol or a mixture thereof at ambient to boiling temperature for 0.5 to 24 hours,
b) lyophilization of the aqueous, alcoholic or hydroalcoholic extract to give a lyophilized powder, and
c) reconstitution of the lyophilized extract in water to achieve a concentration of the triterpenoid saponins that is calculated as a concentration of 4 to 8 % w/w hederagenin.

12. A process according to claim 11, **characterised in that** the alcohol is selected from a C 1-4 alcohol.

13. A process according to claim 11 or 12, **characterised in that** the C1-4 alcohol is methanol, ethanol, n-propanol, iso-propanol, n-butanol, iso-butanol or tert-butanol.

14. Aqueous, alcoholic or hydroalcoholic extract comprising a mixture of triterpenoid saponins derived from the pericarp of the fruit of Sapindus trifoliatus, wherein the triterpenoid saponins contain hederagenin and wherein the concentration of the triterpenoid saponins is calculated as a concentration of 4 to 8 % w/w hederagenin.

15. Extract according to claim 14, **characterised in that** the extract is in the form of a lyophilized powder.

16. A process for the preparation of an anticonvulsant pharmaceutical composition comprising
a) lyophilizing an extract as claimed in claim 14,
b) adding the lyophilized extract to a mixture of Chlorobutanol and Phenylethyl alcohol in water and sodium chloride to get a uniform dispersion,
c) filtering the dispersion,
d) mixing the dispersion with a dispersion of Xanthan gum dissolved in purified water, and
e) adjusting the pH between 4.5 and 6.5.

17. Use of the pharmaceutical composition according to any of claims 1 to 10 for manufacture of a medicament for the prophylactic treatment of migraine by its administration through intra nasal route.

## Patentansprüche

1. Antikonvulsive pharmazeutische Zusammensetzung zur nasalen Verabreichung mit Bindungsaffinitäten für die folgenden Rezeptorstellen, nämlich die GABA-A-Agonisten-Stelle, Glutamat-AMPA-Stelle, Glutamat-Kainat-Stelle, Glutamat-NMDA-Agonisten-Stelle, Glutamat-NMDA-Glycin-Stelle (strychnininsensitiv) und den Natriumkanal (Stelle 2), umfassend
einen wässrigen, alkoholischen oder wässrig-alkoholischen Extrakt, der ein Gemisch von Triterpenoid-Saponinen, gewonnen aus dem Perikarp der Früchte von Sapindus trifoliatus, enthält, und pharmazeutisch annehmbare Zusatzstoffe,
wobei sich die Konzentration der Triterpenoid-Saponine als Hederagenin berechnet, wobei das Hederagenin 0,001 bis 1% Gew./Vol. der antikonvulsiven pharmazeutischen Zusammensetzung ausmacht.

2. Antikonvulsive pharmazeutische Zusammensetzung nach Anspruch 1, **dadurch gekennzeichnet, dass** sich die Konzentration der Triterpenoid-Saponine als eine Konzentration von 0,004 bis 0,08% Gew./Vol. Hederagenin in der pharmazeutischen Zusammensetzung berechnet.

3. Antikonvulsive pharmazeutische Zusammensetzung nach Anspruch 1 oder 2, **dadurch gekennzeichnet, dass** der Extrakt in Form eines lyophilisierten Pulvers vorliegt.

4. Antikonvulsive pharmazeutische Zusammensetzung nach einem der Ansprüche 1 bis 3, **dadurch gekennzeichnet, dass** die pharmazeutisch annehmbaren Zusatzstoffe Mittel zum Einstellen der Tonizität, der Viskosität, des pHs und ein Konservierungsmittel umfassen.

5. Antikonvulsive pharmazeutische Zusammensetzung nach Anspruch 4, **dadurch gekennzeichnet, dass** das Mittel zum Einstellen der Tonizität Natriumchlorid ist.

6. Antikonvulsive pharmazeutische Zusammensetzung nach Anspruch 4 oder 5, **dadurch gekennzeichnet, dass** das Mittel zum Einstellen der Viskosität unter Xanthan-Gummi, Carboxymethylcellulose, Methylcellulose, Hydroxypropylmethylcellulose, Polyvinylpyrrolidon, Polyvinylalkohol und Carbomeren ausgewählt ist.

7. Antikonvulsive pharmazeutische Zusammensetzung nach einem der Ansprüche 4 bis 6, **dadurch gekennzeichnet, dass** das Mittel zum Einstellen des pHs unter Citronensäure, Natriumcitrat, Kaliumdihydrogenphosphat, Essigsäure, Natriumacetat und Ammoniumacetat ausgewählt ist.

8. Antikonvulsive pharmazeutische Zusammensetzung nach einem der Ansprüche 4 bis 7, **dadurch gekennzeichnet, dass** das Konservierungsmittel unter Chlorbutanol, Phenylethylalkohol und Parabenen ausgewählt ist.

9. Antikonvulsive pharmazeutische Zusammensetzung nach einem der Ansprüche 1 bis 8, **dadurch gekennzeichnet, dass** der pH im Bereich von 4,5 bis 6,5 liegt.

10. Antikonvulsive pharmazeutische Zusammensetzung nach einem der Ansprüche 1 bis 9, **dadurch gekennzeichnet, dass** die Zusammensetzung in einer Form vorliegt, die unter Nasentropfen, Nasensprays, Nasenpulvern, halbfesten nasalen Zubereitungen, Nasenspülungen oder Nasenstäbchen ausgewählt ist.

11. Verfahren zur Herstellung eines Extrakts, der ein Gemisch von Triterpenoid-Saponinen enthält, wobei das Verfahren die folgenden Schritte umfasst:
a) Extraktion des Perikarps der Frucht von Sapindus trifoliatus mit Wasser oder einem Alkohol oder einem Gemisch daraus bei Umgebungs- bis Siedetemperatur für 0,5 bis 24 Stunden,
b) Lyophilisierung des wässrigen, alkoholischen oder wässrig-alkoholischen Extrakts, um ein lyophilisiertes Pulver zu erhalten, und
c) Rekonstitution des lyophilisierten Extrakts in Wasser, derart, dass eine Konzentration der Triterpenoid-Saponine erzielt wird, die sich als eine Konzentration von 4 bis 8% Gew./Gew. Hederagenin berechnet.

12. Verfahren nach Anspruch 11, **dadurch gekennzeichnet, dass** der Alkohol unter einem C1-4 Alkohol ausgewählt wird.

13. Verfahren nach Anspruch 11 oder 12, **dadurch gekennzeichnet, dass** der C1-4 Alkohol Methanol, Ethanol, n-Propanol, Isopropanol, n-Butanol, Isobutanol oder tert-Butanol ist.

14. Wässriger, alkoholischer oder wässrig-alkoholischer Extrakt, der ein Gemisch von Triterpenoid-Saponinen, gewonnen aus dem Perikarp der Frucht von Sapindus trifoliatus, umfasst, wobei die Triterpenoid-Saponine Hederagenin enthalten und wobei sich die Konzentration der Triterpenoid-Saponine als eine Konzentration von 4 bis 8% Gew./Gew. Hederagenin berechnet.

15. Extrakt nach Anspruch 14, **dadurch gekennzeichnet, dass** der Extrakt in Form eines lyophilisierten Pulvers vorliegt.

16. Verfahren zur Herstellung einer antikonvulsiven pharmazeutischen Zusammensetzung, umfassend
a) das Lyophilisieren eines Extrakts, wie er in Anspruch 14 beansprucht wird,
b) das Hinzugeben des lyophilisierten Extrakts zu einem Gemisch aus Chlorbutanol und Phenylethylalkohol in Wasser und Natriumchlorid, um eine gleichmäßige Dispersion zu erhalten,
c) das Filtrieren der Dispersion,
d) das Mischen der Dispersion mit einer Dispersion von Xanthan-Gummi, gelöst in gereinigtem Wasser, und
e) das Einstellen des pHs zwischen 4,5 und 6,5.

17. Verwendung der pharmazeutischen Zusammensetzung nach einem der Ansprüche 1 bis 10 zur Herstellung eines Medikaments für die prophylaktische Behandlung von Migräne durch dessen Verabreichung auf intranasalem Weg.

## Revendications

1. Composition pharmaceutique anticonvulsive destinée à l'administration nasale comprenant des affinités de liaison pour les sites récepteurs suivants, à savoir le site agoniste GABA-A, le site glutamate AMPA, le site glutamate kainate, le site agoniste glutamate NMDA, le site glutamate glycine NMDA (insensible à la strychnine) et le canal de sodium (site 2), comportant :
un extrait aqueux, d'alcool ou d'eau et d'alcool qui contient un mélange de saponines triterpénoïdes, gagné à partir du péricarpe des fruits du sapindus trifoliatus, et
des additifs acceptables sur le plan pharmaceutique,
dans laquelle la concentration des saponines triterpénoïdes se calcule comme hédéragénine et dans laquelle l'hédéragénine comporte 0,001 à 1% poids/volume de la composition pharmaceutique anticonvulsive.

2. Composition pharmaceutique anticonvulsive selon la revendication 1, **caractérisée en ce que** la concentration en saponines triterpénoïdes se calcule comme une concentration de 0,004 à 0,08% poids/volume d'hédéragénine dans la composition pharmaceutique.

3. Composition pharmaceutique anticonvulsive selon l'une ou l'autre des revendications 1 et 2, **caractérisée en ce que** l'extrait est présent sous la forme d'une poudre lyophilisée.

4. Composition pharmaceutique anticonvulsive selon l'une des revendications 1 à 3, **caractérisée en ce que** les additifs acceptables sur le plan pharmaceutique comportent des agents pour l'ajustement de la tonicité, de la viscosité, du pH ainsi qu'un agent de conservation.

5. Composition pharmaceutique anticonvulsive selon la revendication 4, **caractérisée en ce que** l'agent pour l'ajustement de la tonicité est du chlorure de sodium.

6. Composition pharmaceutique anticonvulsive selon l'une ou l'autre des revendications 4 et 5, **caractérisée en ce que** l'agent pour l'ajustement de la viscosité est choisi parmi le caoutchouc de xanthène, la cellulose carboxyméthylique, la cellulose méthylique, la cellulose d'hydroxypropyle méthylique, le pyrrolidone polyvinylique, l'alcool polyvinylique et des carbomères.

7. Composition pharmaceutique anticonvulsive selon l'une des revendications 4 à 6, **caractérisée en ce que** l'agent pour l'ajustement du pH est choisi parmi l'acide citrique, le citrate de sodium, le phosphate dihydrogénique de potassium, l'acide acétique, l'acétate de sodium et l'acétate d'ammonium.

8. Composition pharmaceutique anticonvulsive selon l'une des revendications 4 à 7, **caractérisée en ce que** l'agent de conservation est choisi parmi le butanol de chlore, l'alcool phényléthylique et des parabènes.

9. Composition pharmaceutique anticonvulsive selon l'une des revendications 1 à 8, **caractérisée en ce que** le pH se trouve dans la plage de 4,5 à 6,5.

10. Composition pharmaceutique anticonvulsive selon l'une des revendications 1 à 9, **caractérisée en ce que** la composition est présente sous une forme qui est choisie parmi des gouttes pour le nez, des sprays pour le nez, des poudres pour le nez, des préparations nasales semi solides, des rinçages de nez ou des bâtonnets pour le nez.

11. Procédé pour fabriquer un extrait qui contient un mélange de saponines triterpénoïdes, dans lequel le procédé comporte les étapes suivantes :
a) l'extraction du péricarpe du fruit du sapindus trifoliatus avec de l'eau ou un alcool ou un mélange de ceux-ci à une température comprise entre la température ambiante et la température d'ébullition pendant 0,5 à 24 heures,
b) la lyophilisation de l'extrait aqueux, d'alcool ou d'eau et d'alcool pour obtenir une poudre lyophilisée, et
c) la reconstitution de l'extrait lyophilisé dans de l'eau de telle sorte qu'une concentration de saponine triterpénoïde est obtenue, laquelle se calcule comme une concentration de 4 à 8% en poids/poids d'hédéragénine.

12. Procédé selon la revendication 11, **caractérisé en ce que** l'alcool est choisi parmi un alcool C1-4.

13. Procédé selon l'une ou l'autre des revendications 11 et 12, **caractérisé en ce que** l'alcool C1-4 est du méthanol, de l'éthanol, du n-propanol, de l'isopropanol, du n-butanol, de l'isobutanol ou du tert-butanol.

14. Extrait aqueux, d'alcool ou d'eau et d'alcool, lequel comporte un mélange de saponines triterpénoïdes, gagné à partir du péricarpe du fruit du sapindus trifoliatus, dans lequel les saponines triterpénoïdes contiennent de l'hédéragénine et dans lequel la concentration de la saponine triterpénoïde se calcule comme une concentration de 4 à 8% en poids/poids d'hédéragénine.

15. Extrait selon la revendication 14, **caractérisé en ce que** l'extrait est présent sous la forme d'une poudre lyophilisée.

16. Procédé pour fabriquer une composition pharmaceutique anticonvulsive, comportant :
a) la lyophilisation d'un extrait, tel qu'il est revendiqué dans la revendication 14,
b) l'addition de l'extrait lyophilisé pour former un mélange de butanol de chlore et d'alcool phenyléthylique dans de l'eau et dans du chlorure de sodium, dans le but d'obtenir une dispersion régulière,
c) le filtrage de la dispersion,
d) le mélange de la dispersion avec une dispersion de caoutchouc de xanthène, dissoute dans de l'eau épurée, et
e) le réglage du pH entre 4,5 et 6,5.

17. Utilisation de la composition pharmaceutique selon l'une des revendications 1 à 10 afin de fabriquer un médicament pour le traitement prophylactique de la migraine suite à l'administration de celui-ci par voie intra nasale.
